Europäisches Patentamt

European Patent Office

Office européen des brevets

(19)

(11) **EP 1 252 819 A1**

(12) # DEMANDE DE BREVET EUROPEEN

(43) Date de publication:
**30.10.2002 Bulletin 2002/44**

(51) Int Cl.⁷: **A01N 37/16**, A61L 2/18

(21) Numéro de dépôt: **02290459.3**

(22) Date de dépôt: **26.02.2002**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **27.04.2001 FR 0105727**

(71) Demandeur: **SOCIETE D'EXPLOITATION DE PRODUITS POUR LES INDUSTRIES CHIMIQUES, S.E.P.P.I.C.**
**75321 Paris Cédex 07 (FR)**

(72) Inventeurs:
• **Le Rouzic, Daniel**
  **95120 Ermont (FR)**
• **Gamet, Jean-Claude**
  **71460 Saint Martin du Tartre (FR)**
• **Lauriot, Jean-Marc**
  **71150 Demigny (FR)**

(74) Mandataire: **Conan, Philippe Claude et al**
**L'Air Liquide SA,**
**75 Quai d'Orsay**
**75321 Paris Cedex 01 (FR)**

(54) **Composition désinfectante pour les dispositifs ophtalmologiques**

(57) Composition consistant en une solution aqueuse comprenant entre 0,10 % en poids et 0,20 % en poids d'acide peracétique, entre 2 % en poids et 6 % en poids de peroxyde d'hydrogène, entre 2 % en poids et au plus 6 % en poids d'acide acétique, entre 0, % et 0,01 % en poids de benzotriazole pour désinfecter le matériel d'ophtalmologie destiné aux actes non invasifs et plus particulièrement pour désinfecter les tonomètres à aplanation, les prismes de dédoublement ou les verres de contact à trois miroirs.

**EP 1 252 819 A1**

**Description**

L'invention a pour objet un nouveau procédé désinfection de dispositifs médicaux utilisés en ophtalmologie.

L'évolution actuelle des soins médicaux conduit à la multiplication des techniques exploratrices mettant en oeuvre des dispositifs de plus en plus petits, souvent fabriqués avec des matériaux thermosensibles. Il s'ensuit que ces dispositifs, ne peuvent pas être stérilisés à chaud et que, lorsqu'ils ne sont pas à usage unique, ils doivent être nettoyés et désinfectés chimiquement après chaque utilisation. Selon la nature du tissu, avec lequel le dispositif médical entre en contact lors de son utilisation, on établit trois niveaux de risques infectieux, auxquels correspondent des niveaux de traitement de désinfection requis pour un spectre d'activité à atteindre. Au haut niveau de risque infectieux, correspond un traitement de haut niveau qui doit être efficace contre tous les types microorganismes y compris les spores bactériennes.

Une opération de désinfection chimique comprend généralement les étapes suivantes :

a) un pré-traitement, dont le but consiste à faciliter les étapes ultérieures, en empêchant par exemple les salissures de sécher ;

b) un nettoyage, dont l'objectif est d'éliminer les salissures, qui va conjuguer l'action physico-chimique de la solution utilisée et les actions mécaniques effectuées manuellement.

c) la désinfection chimique, impliquant la mise en contact du dispositif à désinfecter avec une solution désinfectante ;

d) le rinçage final, dont l'objectif est d'éliminer tout résidu de produit, tout en évitant les re-contaminations ; ceci implique l'utilisation d'une eau adaptée à cette exigence qui, selon le niveau de risque, sera stérile ou filtrée ; et, lorsque le dispositif n'est pas immédiatement réutilisé ;

e) le séchage, puis

f) Le stockage, ces deux dernières étapes devant, elles aussi, être effectuées dans des conditions évitant les re-contaminations du dispositif.

Lorsque l'instrument à désinfecter ne porte pas de salissures, les étapes a) et b) ou seule l'étape a) peuvent être omises.

Le glutaraldéhyde à 2% dans l'eau, en désinfection manuelle et à 20% dans l'eau en désinfection automatique, est aujourd'hui la matière active la plus utilisée en Europe. Le temps de désinfection efficace avec ce produit est, en désinfection manuelle, compris entre 20 minutes dans le cas d'un risque infectieux médian, et 1 heure dans le cas d'un risque d'infection élevé. Cependant, le glutaraldéhyde est toxique, et les opérations le mettant en oeuvre, en particulier la désinfection manuelle, doivent être réalisées dans des espaces ventilés, voire sous des hottes aspirantes ; de plus il fixe les protéines et est peu efficace contre les spores. En effet, une solution aqueuse de 2% en poids de glutaraldéhyde est inefficace à 20°C après 20 minutes, tant sur Bacillus cereus que sur Bacillus subtilis. Il ne devient efficace contre ces souches qu'après au moins 1 heure de trempage.

C'est pourquoi la demanderesse a recherché un substitut à ce produit ; elle s'est intéressée aux solutions aqueuses d'acide peracétique, qui sont connues pour leur efficacité sur l'ensemble des microorganismes, notamment sur les spores, lorsqu'elles sont à une concentration suffisante en peracide soit à partir d'environ 5000 ppm pour un abattement de 5 log et environ 2500 ppm pour un abattement de 3 log en 5 minutes. Cependant à cette concentration, l'acide peracétique devient corrosif pour certains des matériaux constitutifs Des dispositifs médicaux et notamment pour leurs parties métalliques et il commence aussi à être légèrement irritant sur la peau. On sait aussi, que le peroxyde d'hydrogène seul n'est sporicide qu'à des concentrations minimales de 10% à 20% en poids.

La demanderesse a donc cherché à mettre au point un procédé manuel de désinfection de dispositifs médicaux utilisés en ophtalmologie, mettant en oeuvre l'acide peracétique à des doses non irritantes et non corrosives, qui permette d'obtenir en 20 minutes à 20°C, un abattement logarithmique de 3 log sur Bacillus cereus, soit une réduction de $10^3$ spores par $cm^3$, ce résultat étant déterminé par l'analyse normalisée AFNOR T 72 301. Cela constitue une condition minimale requise pour envisager une exploitation commerciale de ce procédé en remplacement du glutaraldéhyde et du CIDEX™ PA, qui est une solution aqueuse désinfectante comprenant 800 ppm (soit environ 0,08% en poids) d'acide peracétique et 1% en poids de peroxyde d'hydrogène. En effet, la demanderesse a constaté qu'une solution aqueuse comprenant 800 ppm (soit environ 0,08% en poids) d'acide peracétique et 1% en poids de peroxyde d'hydrogène, après un an de stockage et 1 semaine d'utilisation était inefficace contre Bacillus cereus à 20°C après 20 minutes. Le choix de l'activité sporicide comme indice d'efficacité de l'acide peracétique est dicté par le haut niveau de désinfection exigé. c'est elle qui généralement requiert la dose la plus élevée en désinfectant, à cause de la présence d'une tunique sporale autour des bactéries augmentant leur résistance. Le choix de Bacillus cereus, comme cible d'étude parmi les souches indiquées dans la norme AFNOR T 72 301, à savoir Bacillus cereus CIP 7803, Bacillus subtilis et Clostridium sporogenes CIP 7939, comme cible de l'activité sporicide de l'acide peracétique, découle du résultat d'un test préliminaire, au cours duquel la demanderesse s'est aperçue que Bacillus cereus était plus facile à

détruire que <u>Bacillus subtilis</u> avec du glutaraldéhyde, alors qu'au contraire <u>Bacillus cereus</u> était plus difficile à détruire que <u>Bacillus subtilis</u> avec de l'acide peracétique.

La demanderesse a trouvé de façon inattendue que l'acide peracétique est très efficace pour désinfecter le matériel d'ophtalmologie destiné aux actes non invasifs sans induire d'irritation cutanée, de conjonctivite ou ni de kératite. Elle a de plus trouvé que la présence conjointe d'acide peracétique et de benzotriazole, composé pourtant connu pour être sensible aux milieux oxydants, permettait d'obtenir une composition biocide efficace, plus stable dans le temps et non corrosive à l'encontre du matériel d'ophtalmologie.

L'invention a pour objet une composition consistant en une solution aqueuse comprenant entre 0,05 % en poids et 0,20 % en poids d'acide peracétique, entre 2 % en poids et 6 % en poids de peroxyde d'hydrogène, entre 2 % en poids et au plus 6 % en poids d'acide acétique, et de 0% en poids à 0,1 % en poids de benzotriazole.

Par 0 % en poids on indique dans la définition précédente, que la composition objet de la présente invention peut ne pas contenir de benzotriazole.

Selon un aspect préféré de la présente invention, la composition telle que définie ci- dessus contient du benzotriazole et plus particulièrement en contient entre 0,001 % et 0,1% en poids.

Selon un aspect particulier de la présente invention, la composition telle que définie ci- dessus contient entre 0,10% en poids et 0,15% en poids d'acide peracétique.

La solution aqueuse définie ci-dessus, comprend éventuellement en outre, un ou plusieurs additifs choisis notamment parmi les agents tensioactifs non ioniques, les oxydes d'amines, les inhibiteurs de corrosion, les agents stabilisants, les acides forts, les colorants ou les parfums.

Comme agents tensioactifs non ioniques, on désigne dans la présente demande de brevet, ceux décrits dans la demande de brevet européen publiée sous le numéro EP 0 873 687, plus particulièrement, les produits commerciaux suivants : le GENAPOL™ 2822, le GENAPOL™ 2908, le GENAPOL™ 2908D, le GENAPOL™ 2909, le TRITON™ DF12, le TRITON™ DF16, le TRITON™ CF10, le DOWFAX™ 20B102, l'AKYPO™ RO 90, le SYNPERONIC™ LF RA 30 ou le SIMULSOL™ NW 900, l'AKYPO LF2 ou l'AKYPO LF4. Ces produits, disponibles dans le commerce, ont la composition chimique suivante :

| nom commercial | composition chimique |
| --- | --- |
| GENAPOL™ 2822 | Mélange d'alcools alkoxylés en $C_{10}$, $C_{12}$, $C_{14}$ (5OE, 4OP) |
| GENAPOL™ 2908D | Mélange d'alcools alkoxylés en $C_{11}$, $C_{13}$, $C_{15}$ (6 à 7OE, 3OP) |
| GENAPOL™ 2909 | Mélange d'alcools alkoxylés en $C_{12}$, $C_{14}$ (5OE, 3OP) |
| TRITON™ DF12 | Ethers benzyliques d'alcools alkoxylés en $C_8$, $C_{10}$ (2OE, 5OP) |
| TRITON™ DF16 | Mélange d'alcools alkoxylés en $C_8$, $C_{10}$ (6OE, 3OP) |
| TRITON™ CF10 | Ether benzylique d'alcools éthoxylés en $C_8$ (16OE) |
| AKYPO™ RO 90 | Mélange d'éthers carboxyliques éthoxylés en $C_{16}$ $C_{18}$ (9 OE) |
| DOWFAX™ 20B102 | |
| SIMULSOL™ NW 900 | Mélange d'alcools alkoxylés (x OE, y OP) |
| AKYPO LF2 | Ether carboxylique éthoxylé en $C_8$ (8 OE) |
| AKYPO LF4 | Mélange d'éthers carboxyliques éthoxylés en $C_6$, $C_8$ (7 OE) |

Comme agents tensioactifs non-ioniques, on préfère ceux correspondant à la formule (I) suivante :

$$R_1\text{-O-}[CH(R_2)\text{-}CH(R_3)\text{-O}]_n\text{-}R_4 \tag{I}$$

dans laquelle $R_1$ représente un radical hydrocarboné linéaire ou ramifié, saturé ou insaturé comprenant de 5 à 31 atomes de carbones, $R_2$ et $R_3$ représentent indépendamment l'un de l'autre, un atome d'hydrogène ou un radical alkyle comprenant 1 ou 2 atomes de carbone, $R_4$ représente un atome d'hydrogène, un radical alkyle linéaire ou ramifié comprenant de 1 à 4 atomes de carbone ou un radical benzyle, et n représente un nombre entier compris entre 1 et 50 et de préférence inférieur à 20.

Par radical hydrocarboné linéaire ou ramifié, saturé ou insaturé, comprenant de 5 à 31 atomes de carbone, on désigne pour $R_1$ dans la formule (I) telle que définie précédemment notamment les radicaux alkyles ou les radicaux alkènyle. $R_1$ représente plus particulièrement un radical choisi parmi les radicaux hexyle, heptyle, octyle, nonyle, décyle, undécyle, undécènyle, dodécyle, tridécyle, tetradécyle, pentadécyle, hexadécyle, heptadécyle, octadécyle, octadécènyle, octadécatriènyle, octadécadiènyle, eicosanyle ou béhènyle. lesdits radicaux étant linéaires ou ramifiés.

Selon un aspect particulier de la présente invention, $R_1$ représente un radical choisi de préférence parmi les radicaux alkyles linéaires ou ramifiés comportant de 8 à 18 atomes de carbone.

Par radical alkyle comprenant de 1 à 4 atomes de carbone, on désigne pour $R_4$ dans la formule (I) telle que définie précédemment, les radicaux méthyle, éthyle, propyle ou butyle, linéaires ou ramifiés.

Le groupe divalent radical $-[CH(R_2)-CH(R_3)-O]_n-$ représente, soit une chaîne composée uniquement de groupes éthoxyle ($R_2$ et $R_3$ = H), soit une chaîne composée uniquement de groupes propoxyle ($R_2$ ou $R_3$ = $CH_3$), soit une chaîne composée uniquement de groupes butoxyle ($R_2$ et $R_3$ = $CH_3$) soit une chaîne composée d'un mélange de deux sortes ou des trois sortes de groupes énoncés ci-dessus. Dans ce dernier cas, les fragments $-CH_2-CH_2-O-$, $-CH(CH_3)-CH_2-O-$ et $-CH(CH_3)-CH(CH_3)-O-$, sont distribués dans ladite chaîne, de façon séquencée ou aléatoire.

La solution aqueuse d'acide peracétique objet de la présente invention, peut contenir jusqu'à 0,1% en poids d'au moins un agent tensioactif non ionique.

Comme oxyde d'amine, on désigne dans la présente demande de brevet, ceux décrits dans la demande de brevet européen publiée sous le numéro EP 0 873 687 et notamment ceux correspondant à la formule (II) :

$$(R_5)(R_6)(R_7) \text{ N -> O} \qquad\qquad (II).$$

dans laquelle $R_5$ représente un radical hydrocarboné linéaire ou ramifié, saturé ou insaturé comprenant de 8 à 18 atomes de carbones, $R_6$ et $R_7$ représentent chacun, un radical méthyle et plus particulièrement, ceux commercialisés sous la marque AROMOX™ et notamment l'AROMOX™ MCD-W, dont le principe actif est le N-oxyde de cocodiméthylamine . La solution aqueuse d'acide peracétique mise en oeuvre dans le procédé objet de la présente invention, peut contenir jusqu'à 0,1% en poids d'au moins un oxyde d'amine.

La solution aqueuse d'acide peracétique mise en oeuvre dans le procédé objet de la présente invention, peut contenir jusqu'à 0,7% en poids d'un autre inhibiteur de corrosion choisi de préférence entre le dihydrogénophosphate de sodium, $NaH_2PO_4$ et le phosphate disodique, $Na_2HPO_4$.

La solution aqueuse d'acide peracétique mise en oeuvre dans le procédé objet de la présente invention, peut contenir jusqu'à 0,1% en poids d'un agent stabilisant, qui est de préférence le pyrophosphate de sodium.

Comme agents colorants stables au contact de l'acide peracétique, on désigne notamment ceux décrits dans la demande de brevet européen publiée sous le numéro EP 0 658 309.

La solution aqueuse d'acide peracétique mise en oeuvre dans le procédé objet de la présente invention, peut contenir jusqu'à 0,03% en poids d'un agent colorant.

Comme parfums stables au contact de l'acide peracétique, on désigne notamment ceux décrits dans la demande de brevet européen publiée sous le numéro EP 0 596 493.

La solution aqueuse d'acide peracétique mise en oeuvre dans le procédé objet de la présente invention, peut contenir jusqu'à 0,03% en poids de parfum.

Comme exemple de composition prête à l'emploi particulièrement approprié à la désinfection par trempage manuel, il y a une composition telle que définie précédemment, sous forme d'une solution aqueuse, dans de l'eau déminéralisée, comprenant :

de 0,10% à 0,12% en poids d'acide peracétique,
de 2,5% à 3,5% en poids de peroxyde d'hydrogène,
de 2% à 4% en poids d'acide acétique,
de 0,001 % à 0,002% en poids de GENAPOL™ 2908D,
de 1% à 2% en poids de dihydrogénophosphate de sodium ($12H_2O$),
de 0,01% à 0,03% en poids de pyrophosphate de sodium,
de 0,01% à 0,05% en poids de benzotriazole et
de l'eau pour compléter à 100% en poids..

Cette composition peut éventuellement contenir aussi de 0,01% à 0,03% en poids de N-oxyde de cocodiméthylamine et/ou jusqu'à 0,003% en poids d'un agent colorant, tel que par exemple l'ORANGE SOLEIL W200™.

L'invention a aussi pour objet, l'utilisation d'une composition telle que définie précédemment, pour désinfecter le matériel d'ophtalmologie destiné aux actes non invasifs.

Par matériel d'ophtalmologie destiné aux actes non invasifs, on entend plus particulièrement, les tonomètres à aplanation, les prismes de dédoublement ou les verres de contact à trois miroirs ou encore les pinces ophtalmologiques.

L'invention a aussi pour objet un procédé manuel de désinfection de matériel d'ophtalmologie destiné aux actes non invasifs, caractérisé en ce qu'il comprend une étape au cours de laquelle ledit matériel est immergé dans la composition telle que définie précédemment.

L'invention a plus particulièrement pour objet, un procédé de désinfection, tel que défini précédemment, dans lequel l'étape de mise en contact dudit matériel avec ladite solution est suivie d'une étape de rinçage puis, si nécessaire, d'une étape de séchage.

L'étape de rinçage final a pour objectifs multiples, d'éliminer tout résidu de produit tout en évitant les re-contaminations, de diminuer, voire d'éliminer le risque de provoquer une conjonctivite ou une kératite chez le patient suivant ; ceci implique l'utilisation d'une eau adaptée à cette exigence qui, selon le niveau de risque, sera stérile ou filtrée ; le séchage est nécessaire lorsque le dispositif n'est pas immédiatement réutilisé. Le séchage peut consister en l'essuyage des dispositifs au moyen de lingettes stériles.

Selon un mode tout particulier du procédé tel que défini ci-dessus le matériel est essuyé après être sorti du bain de désinfection, notamment avec un tissu absorbant de préférence stérile, au moyen, puis est rincé à l'eau et si désiré essuyé une nouvelle fois.

Il est bien entendu que la présente invention inclut aussi le procédé de désinfection de plusieurs dispositifs à la fois.

Le procédé est mis en oeuvre à température ambiante, soit environ entre 15 et 35°C, généralement autour de 20 à 25°C, et le trempage dure de 5 à 20 minutes, souvent autour de 10 minutes.

## Exemple

### a) Activité biocide de la composition selon l'invention Trempage manuel

On détermine les activités bactéricide, fongicide et sporicide d'une solution A, comprenant à t = 0, de 0,10% à 0,12% en poids d'acide peracétique,

de 3 % à 3,5 % en poids de peroxyde d'hydrogène,
de 2 % à 4 % en poids d'acide acétique,
de 0,001% à 0,002% en poids de GENAPOL™ 2908D,
de 1 % à 2 % en poids de dihydrogénophosphate de sodium (12H$_2$O),
de 0,01% à 0,03% en poids de pyrophosphate de sodium,
de 0,03 % à 0,05% en poids de benzotriazole en mettant en oeuvre les méthodes d'analyse décrites respectivement dans les normes EN 1040 (activité bactéricide), EN 1275 (activité fongicide), AFNOR 72180 (activité virucide) et AFNOR T 72301 (activité sporicide).

On constate des activités bactéricide, et fongicide similaires pour les deux solutions après un temps de contact défini par la norme considérée ; la détermination de l'activité sporicide conduit aux résultats recensés dans le tableau suivant (temps de contact : 20 minutes ; température : 20°C ; abattement en spores/ml):

| SOUCHES | glutaraldéhyde à 2% dans l'eau | SOLUTION A | SOLUTION B |
|---|---|---|---|
| Bacillus cereus | inefficace après 20 minutes | $10^3$ après 20 minutes | < $10^3$ après 20 minutes |
| Bacillus Subtilis | inefficace après 20 minutes | $10^5$ après 20 minutes | |

On constate donc que la solution A obéit à l'exigence imposée en matière d'activité sporicide sur Bacillus cereus.

### b) Evaluation de la compatibilité des matériels d'ophtalmologie avec la solution A.

On a immergé dans une solution A et dans une solution B contenant à t = 0, de 0,10% à 0,12% en poids d'acide peracétique,

de 3 % à 3,5 % en poids de peroxyde d'hydrogène,
de 2 % à 4 % en poids d'acide acétique,
de 0,001% à 0,002% en poids de GENAPOL™ 2908D,
de 1 % à 2 % en poids de dihydrogénophosphate de sodium (12H$_2$O) et
de 0,01% à 0,03% en poids de pyrophosphate de sodium.

un verre de contact à miroir (échantillons A1 et B1), une pince ophtalmologique de couleur bleutée (échantillons A2 et B2) et un prisme de dédoublement (échantillons A3 et B3).

Après un mois de stockage, on constaté que les titres en acide peracétique dans les trois échantillons de solution B1, B2 et B3, étaient plus faibles que ceux dans les trois échantillons A1, A2 et A3.

Après deux mois de trempage, on a constaté que la pince ophtalmologique immergée de l'échantillon B3 avait changé de couleur et virée au gris, alors que celle de l'échantillon A3 avait conservé sa couleur bleue d'origine.,

|  | Solution A (invention) | | | Solution B (état de la technique) | | |
|---|---|---|---|---|---|---|
|  | A1 | A2 | A3 | B1 | B2 | A3 |
|  | verre | pince | prisme | verre | pince | prisme |
| t = 0 [Acide peracétique] | 1077 ppm | 1077 ppm | 1077 ppm | 1007 ppm | 1007 ppm | 1007 ppm |
| t = 1 mois [Acide peracétique] | 1044 ppm | 1000 ppm | 1106 ppm | 912 ppm | 539 ppm | 995 ppm |

Ceci démontre l'intérêt de la présence de benzotriazole au côté de l'acide peracétique.

**Revendications**

1. Composition consistant en une solution aqueuse comprenant entre 0,05 % en poids et 0,20 % en poids d'acide peracétique, entre 2 % en poids et 6 % en poids de peroxyde d'hydrogène, entre 2 % en poids et au plus 6 % en poids d'acide acétique, et de 0% en poids à 0,1 % en poids de benzotriazole.

2. Composition telle que définie à la revendication 1, **caractérisée en ce qu'**elle contient du benzotriazole et plus particulièrement **en ce qu'**elle contient entre 0,001 % et 0,1% en poids de benzotriazole.

3. Composition telle que définie à l'une quelconque des revendications 1 ou 2, **caractérisée en ce qu'**elle contient entre 0,10% en poids et 0,15% en poids d'acide peracétique.

4. Composition telle que définie à l'une quelconque des revendications 1 à 3, comprenant en outre un ou plusieurs additifs choisis parmi les agents tensioactifs non ioniques, les oxydes d'amines, les inhibiteurs de corrosion, les agents stabilisants, les acides forts, les agents colorants ou les parfums.

5. Composition telle que définie à l'une quelconque des revendications 1 à 4, dans laquelle les agents tensioactifs non-ioniques, sont de formule (I)

$$R_1\text{-O-}[CH(R_2)\text{-}CH(R_3)\text{-O}]_n\text{-}R_4 \qquad\qquad (I)$$

dans laquelle $R_1$ représente un radical hydrocarboné linéaire ou ramifié, saturé ou insaturé comprenant de 5 à 31 atomes de carbones, $R_2$ et $R_3$ représentent indépendamment l'un de l'autre, un atome d'hydrogène ou un radical alkyle comprenant 1 ou 2 atomes de carbone, $R_4$ représente un atome d'hydrogène, un radical alkyle linéaire ou ramifié comprenant de 1 à 4 atomes de carbone ou un radical benzyle, et n représente un nombre entier compris entre 1 et 50 et de préférence inférieur à 20.

6. Composition telle que définie à la revendication 5, pour laquelle dans la formule (I), $R_1$ représente un radical choisi parmi les radicaux hexyle, heptyle, octyle, nonyle, décyle, undécyle, undécènyle, dodécyle, tridécyle, tetradécyle, pentadécyle, hexadécyle, heptadécyle, octadécyle, octadécènyle, octadécatriènyle, octadécadiènyle, eicosanyle ou béhènyle, lesdits radicaux étant linéaires ou ramifiés.

7. Composition telle que définie à l'une quelconque des revendications 5 ou 6, pour laquelle dans la formule (I), $R_4$ représente un radical choisi parmi les radicaux méthyle, éthyle, propyle ou butyle, linéaires ou ramifiés.

8. Composition telle que définie à l'une quelconque des revendications 4 à 7, comprenant jusqu'à 0,1% en poids d'au moins un agent tensioactif non ionique.

**9.** Composition telle que définie à l'une quelconque des revendications 3 à 8, comprenant en outre un oxyde d'amine de formule (II):

$$(R_5)(R_6)(R_7) N -> O \qquad (II).$$

dans laquelle $R_5$ représente un radical hydrocarboné linéaire ou ramifié, saturé ou insaturé comprenant de 8 à 18 atomes de carbones, $R_6$ et $R_7$ représentent chacun, un radical méthyle.

**10.** Composition telle que définie à l'une quelconque des revendications 4 à 9, comprenant jusqu'à 0,1% en poids d'au moins un oxyde d'amine.

**11.** Composition telle que définie à l'une quelconque des revendications 4 à 10, comprenant jusqu'à 0,7% en poids d'un autre inhibiteur de corrosion choisi de préférence entre le dihydrogénophosphate de sodium, $NaH_2PO_4$ et le phosphate disodique, $Na_2HPO_4$.

**12.** Composition telle que définie à l'une quelconque des revendications 4 à 11, comprenant jusqu'à 0,1% en poids d'un agent stabilisant, qui est de préférence le pyrophosphate de sodium.

**13.** Composition telle que définie à l'une quelconque des revendications 4 à 12, comprenant jusqu'à 0,03% en poids d'un agent colorant.

**14.** Composition telle que définie à l'une quelconque des revendications 4 à 13, comprenant jusqu'à 0,03% en poids de parfum.

**15.** Composition telle que définie à l'une des revendications 1 à 14, sous forme d'une solution aqueuse, dans de l'eau déminéralisée, comprenant :

de 0,10% à 0,12% en poids d'acide peracétique,
de 2,5% à 3,5% en poids de peroxyde d'hydrogène,
de 2% à 4% en poids d'acide acétique,
de 0,001% à 0,002% en poids de GENAPOL™ 2908D,
de 1% à 2% en poids de dihydrogénophosphate de sodium ($12H_2O$),
de 0,01% à 0,03% en poids de pyrophosphate de sodium et
de 0,01% à 0,05% en poids de benzotriazole et
de l'eau pour compléter à 100% en poids..

**16.** Composition telle que définie à la revendication 15, contenant en outre de 0,01% à 0,03% en poids de N-oxyde de cocodiméthylamine et/ou de 0% à 0,003% en poids d'un agent colorant, et plus particulièrement l'ORANGE SOLEIL W200™.

**17.** Utilisation d'une composition telle que définie à l'une revendications 1 à 16, pour désinfecter le matériel d'ophtalmologie destiné aux actes non invasifs et plus particulièrement pour désinfecter les tonomètres à aplanation, les prismes de dédoublement ou les verres de contact à trois miroirs.

**18.** Procédé de désinfection de matériel d'ophtalmologie destiné aux actes non invasifs et plus particulièrement de tonomètres à aplanation, de prismes de dédoublement ou de verres de contact à trois miroirs, comprenant une étape au cours de laquelle ledit matériel est immergé dans la composition telle qu'elle est définie à l'une revendications 1 à 16.

**19.** Procédé de désinfection tel que défini à la revendication 18, dans lequel l'étape d'immersion dudit matériel avec ladite solution est suivie d'une étape de rinçage puis, si nécessaire, d'une étape de séchage.

**20.** Variante du procédé tel que défini à la revendication 19, dans laquelle ledit matériel est essuyé après être sorti du bain de désinfection, notamment avec un tissu absorbant de préférence stérile, au moyen, puis est rincé à l'eau puis est si désiré, essuyé une nouvelle fois.

**Office européen des brevets**

# RAPPORT DE RECHERCHE EUROPEENNE

Numéro de la demande

EP 02 29 0459

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X,D | EP 0 873 687 A (CHEMOXAL SA ;SEPPIC SA (FR)) 28 octobre 1998 (1998-10-28) <br> * page 6, ligne 6-16 * <br> * page 6, ligne 50 - ligne 58 * | 1-20 | A01N37/16 <br> A61L2/18 |
| A | WO 98 11777 A (COTTRELL LTD) 26 mars 1998 (1998-03-26) <br> * revendications 1-3 * | 1,2,15 | |
| A | WO 01 19414 A (METREX RES CORP) 22 mars 2001 (2001-03-22) <br> * page 29; tableau 4 * | 1,2,15 | |
| A | FR 2 759 911 A (CHEMOXAL SA) 28 août 1998 (1998-08-28) <br> * revendications * <br> * page 6; tableau * | 1,5-8,15 | |

|  |
|---|
| **DOMAINES TECHNIQUES RECHERCHES (Int.Cl.7)** |
| A01N <br> A61L |

Le présent rapport a été établi pour toutes les revendications

| Lieu de la recherche | Date d'achèvement de la recherche | Examinateur |
|---|---|---|
| LA HAYE | 1 août 2002 | Decorte, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 02 29 0459

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

01-08-2002

| Document brevet cité au rapport de recherche | | Date de publication | Membre(s) de la famille de brevet(s) | | Date de publication |
|---|---|---|---|---|---|
| EP 0873687 | A | 28-10-1998 | FR | 2762516 A1 | 30-10-1998 |
| | | | FR | 2772620 A1 | 25-06-1999 |
| | | | DE | 69805697 D1 | 11-07-2002 |
| | | | EP | 0873687 A1 | 28-10-1998 |
| | | | JP | 11116990 A | 27-04-1999 |
| | | | US | 6168808 B1 | 02-01-2001 |
| WO 9811777 | A | 26-03-1998 | AU | 4419597 A | 14-04-1998 |
| | | | BR | 9712070 A | 15-01-2002 |
| | | | EP | 0957683 A1 | 24-11-1999 |
| | | | JP | 2001506971 T | 29-05-2001 |
| | | | US | 5900256 A | 04-05-1999 |
| | | | WO | 9811777 A1 | 26-03-1998 |
| WO 0119414 | A | 22-03-2001 | AU | 7489400 A | 17-04-2001 |
| | | | WO | 0119414 A1 | 22-03-2001 |
| FR 2759911 | A | 28-08-1998 | FR | 2759911 A1 | 28-08-1998 |
| | | | EP | 0971584 A1 | 19-01-2000 |
| | | | WO | 9837762 A1 | 03-09-1998 |

EPO FORM P0460

Pour tout renseignement concernant cette annexe : voir Journal Officiel de l'Office européen des brevets, No.12/82